(19) Europäisches Patentamt
European Patent Office
Office européen des brevets

(11) Veröffentlichungsnummer: **0 268 133**
**A2**

(12) **EUROPÄISCHE PATENTANMELDUNG**

(21) Anmeldenummer: 87116131.1

(51) Int. Cl.⁴: **C12Q 1/68**

(22) Anmeldetag: 03.11.87

(30) Priorität: 15.11.86 DE 3639109

(43) Veröffentlichungstag der Anmeldung:
25.05.88 Patentblatt 88/21

(84) Benannte Vertragsstaaten:
**BE CH DE FR GB IT LI NL**

(71) Anmelder: **Merck Patent Gesellschaft mit beschränkter Haftung**
**Frankfurter Strasse 250**
**D-6100 Darmstadt(DE)**

(72) Erfinder: **Reckmann, Bernd, Dr.**
**Freiligrathstrasse 7**
**D-6104 Seeheim(DE)**
Erfinder: **Rieke, Erwin, Dr.**
**Hermannstrasse 12**
**D-6104 Seeheim(DE)**

(54) **Verfahren zur Bestimmung von Nucleinsäuren.**

(57) Die Erfindung betrifft ein Verfahren zur Bestimmung von Nucleinsäuren in einer biologischen Probe durch Überführung der zu bestimmenden Nucleinsäuresequenzen in Einzelstränge und Umsetzung dieser Einzelstränge mit einer Polynucleotidsonde. Die beim Kontakt der Polynucleotidsonde, bestehend aus einem zur nachzuweisenden Nucleinsäure komplementären Bereich und einem Indikatorbereich, mit der nachzuweisenden Nucleinsäurespezies unter Hybridisierungsbedingungen entstehende Konformationsänderung im Indikatorbereich wird nachgewiesen.

## Verfahren zur Bestimmung von Nucleinsäuren

Die Erfindung betrifft ein Verfahren zur Bestimmung von Nucleinsäuresequenzen in einer biologischen Probe und Auswertung über eine Konformationsänderung.

Die Verfügbarkeit einer immer größer werdenden Zahl von clonierten Genen, verbunden mit der hohen Präzision der DNA-Hybridisierung, eröffnet der medizinischen Diagnostik neue Möglichkeiten. So können eine ganze Reihe von Erbkrankheiten mit Hilfe der molekularen Hybridisierung pränatal diagnostiziert werden, was bisher nicht möglich war, weil das (veränderte) Genprodukt nicht nachweisbar oder unbekannt war oder die DNA-Modifikation in nicht-codierten Regionen stattfand. Aber auch bei der Bestimmung von Pathogenen bei Patienten mit akuten Infektionen gewinnt die DNA-Diagnostik immer mehr an Bedeutung. So ist zum Beispiel nur durch die DNA-Analyse ein direkter und schneller Nachweis bestimmter Viren möglich.

Bestimmungen von Pathogenen und von genetischen Defekten werden bisher nur in wenigen klinischen Laboratorien und Laborpraxen durchgeführt, weil die molekulare Hybridisierung im Vergleich zu den klassischen immunologischen Methoden (RIA, ELISA) viel aufwendiger ist und mehr Sachwissen voraussetzt. Es besteht deshalb ein dringendes Bedürfnis, die DNA-Diagnostik so zu verbessern, daß die Hybridisierungsmethoden entscheidend vereinfacht werden.

Es sind bereits Methoden zur Bestimmung von DNA bekannt, wobei die Auswertung über die Markierung mit Radioisotopen, mit Enzymen und fluoreszierenden oder chemilumineszierenden Molekülen erfolgt. Aus der GB 2 125 964 ist z.B. ein Verfahren zur Nucleinsäurebestimmung bekannt, bei dem die zu bestimmende Nucleinsäure in Einzelstränge überführt und auf einer Oberfläche fixiert wird; unter Hybridisierungsbedingungen werden die fixierten Einzelstränge mit Sonden behandelt, die eine den Nucleinsäuren der Einzelstränge komplementäre DNA-Sequenz und eine Proteinbindungs-Sequenz haben; die Doppelstrangbildung wird über ein Protein, das an die Proteinbindungs-Sequenz bindet, ausgewertet. In der EP-OS 139 489 ist eine Sandwich-Hybridisierungsmethode zur Nucleinsäurebestimmung beschrieben, bei der eine biotinylierte DNA-Sonde, die spezifisch für einen bestimmten Teil eines zu bestimmenden Nucleinsäurestranges ist, an mit Avidin überzogene Mikropartikel gebunden wird. Der markierte Einzelstrang wird mit den präparierten Mikropartikeln gemischt, wobei die Bindung des Einzelstrangs an die Mikropartikel über die biotinylierte DNA-Sonde erfolgt; nach Abtrennung von ungebundenem Material wird die gebundene Nucleinsäure über eine zweite DNA-Sonde mit einem Marker bestimmt.

Aus der EP-OS 164 876 sind Verfahren zur Nucleinsäurebestimmung bekannt, die mit Hilfe eines Reagenzkomplexes durchgeführt werden, der aus einer Polynucleotidsonde, die über Wasserstoffbindungen von Purin/Pyrimidin-Basen mit zu bestimmenden Nucleinsäure hybridisieren kann, und einem markierten Polynucleotid, das über die gleiche Basenpaarbindung mit der Polynucleotidprobe verbunden ist, besteht. Das markierte Polynucleotid ist in einem Abschnitt der Polynucleotidsonde gebunden, die mindestens teilweise mit dem Abschnitt der Polynucleotidsonde übereinstimmt, die mit der zu bestimmenden Nucleinsäure hybridisiert. In Gegenwart von Proteinen, welche die Hybridisierung beschleunigen, und geeigneten Cofaktoren wird das markierte Polynucleotid abgespalten und anschließend nach bekannten Methoden bestimmt.

Die bekannten Methoden haben eine Reihe von Nachteilen:

Hybridisierungsassays werden in der Regel heterogen durchgeführt, wobei die zu bestimmende Nucleinsäure an eine feste Phase gebunden ist. Durch diese Immobilisierung wird einerseits der Anteil der zur Hybridisierung fähigen Nucleinsäuren drastisch erniedrigt und andererseits die Kinetik der Hybridisierungsreaktion stark verschlechtert; außerdem müssen überschüssige DNA-Sonden durch Waschschritte entfernt werden, weil eine Bindung des Proteins auch an die Sonde allein möglich ist. Das Verfahren nach der EP-OS 139 489 erlaubt zwar eine Hybridisierung in Lösung, allerdings muß die nachzuweisende Nucleinsäure gleichzeitig mit zwei DNA-Sonden hybridisieren, was zwangsläufig die Ausbeute erniedrigt und damit zu einer deutlich niedrigeren Empfindlichkeit des Verfahrens führt. Auch mit dem in der EP-OS 164 876 beschriebenen Verfahren kann keine ausreichende Empfindlichkeit erzielt werden, weil das freigesetzte Polynucleotid mit der nachzuweisenden DNA um die eigentliche DNA-Sonde kompetitiert; ferner geht ein großer Teil des freigesetzten Polynucleotids verloren, weil es an den komplementären Strang der nachzuweisenden DNA bindet oder aufgrund seiner geringen Länge unspezifisch mit der nachzuweisenden DNA hybridisiert.

Der Erfindung liegt die Aufgabe zugrunde, ein Verfahren zur Bestimmung von Nucleinsäuresequenzen zur Verfügung zu stellen, das es ermöglicht, die Hybridisierungsreaktion nicht nur sehr viel einfacher als bisher durchzuführen, sondern auch die Empfindlichkeit deutlich zu steigern und die Störanfälligkeit des Verfahrens erheblich zu erniedrigen.

Das Verfahren beruht auf der überraschenden Beobachtung, daß eine Polynucleotidsonde durch Hybridisierung mit einer zu einem Teil dieser Sonde komplementären einzelsträngigen Nucleinsäuresequenz eine neue stabile Konformation einnimmt. Unter Ausnutzung dieser Konformationsänderung der Polynucleotidsonde kann die Nucleinsäurespecies nachgewiesen werden.

Gegenstand der Erfindung ist ein Verfahren zur Bestimmung von Nucleinsäuren in einer biologischen Probe durch Überführung der zu bestimmenden Nucleinsäuresequenzen in Einzelstränge und Umsetzung dieser Einzelstränge mit einer Polynucleotidsonde, das dadurch gekennzeichnet ist, daß die beim Kontakt der Polynucleotidsonde, bestehend aus einem zur nachzuweisenden Nucleinsäure komplementären Bereich und einem Indikatorbereich, mit der nachzuweisenden Nucleinsäurespezies unter Hybridisierungsbedingungen entstehende Konformationsänderung im Indikatorbereich nachgewiesen wird.

Überraschenderweise hat sich gezeigt, daß sich mit dem erfindungsgemäßen Verfahren auf einfache Weise und ohne die üblichen Wasch-und Trennschritte Analysenergebnisse erzielen lassen, die den bisher bekannten Nucleinsäuresequenzbestimmungen mindestens ebenbürtig sind.

Um Nucleinsäuresequenzen von Geweben zu analysieren, wird die DNA (RNA) nach den üblichen Verfahren, wie Proteinase K-Behandlung oder Chloroform-Phenol-Extraktion, isoliert. Handelt es sich bei den zu untersuchenden biologischen Proben um Abstriche oder Seren, ist eine Nucleinsäure-Isolierung nicht notwendig. Die DNA (RNA) muß lediglich in Lösung gebracht werden, was durch Proteinase-oder/und Alkali-Behandlung möglich ist.

Die DNA wird entweder durch Alkalibehandlung (pH 13) und anschließende Neutralisierung oder durch Erhitzen über den Schmelzpunkt der DNA (ca. 70 °C in 50 % Formamid) und schnelles Abkühlen auf die Hybridisierungstemperatur in ihre Einzelstränge zerlegt.

Nach dem erfindungsgemäßen Verfahren besteht die eingesetzte Polynucleotidsonde aus einem zur nachzuweisenden Nucleinsäurespecies komplementären Bereich und aus einem in einer bestimmten Konformation vorliegenden zweiten Bereich, dem sogenannten Indikatorbereich. Dieser Indikatorbereich kann mit dem komplementären Bereich überlappen, nicht überlappen oder durch einen Spacer getrennt sein. Durch Zusammenbringen der Polynucleotidsonde mit der zuvor in Einzelstränge überführten zu bestimmenden Nucleinsäure unter Hybridisierungsbedingungen werden Wechselwirkungen zwischen dem komplementären Bereich und dem Indikatorbereich der Polynucleotidsonde verändert, so daß der Indikatorbereich nach Hybridisierung des komplementären Bereiches mit der zu bestimmenden Nucleinsäurespecies in einer anderen Konformation vorliegt. Die Hybridisierungsbedingungen, d.h. die Dauer und die Temperatur, unter denen die Polynucleotidsonde und die nachzuweisende DNA zur Ausbildung eines Doppelstrangs inkubiert werden müssen, hängt von der Länge und der Komplexität der eingesetzten Polynucleotidsonde ab. Die Temperatur liegt im allgemeinen etwa 5 bis 25 °C unter der Dissoziationstemperatur, die Dauer der Inkubation liegt zwischen etwa 10 Minuten und 12 Stunden.

Durch Hybridisierung mit der zu bestimmenden Nucleinsäure kann sowohl eine Konformationsänderung des Indikatorbereiches der Polynucleotidsonde von einzelsträngiger DNA oder RNA in doppelsträngige DNA oder RNA als auch von doppelsträngiger DNA oder RNA in einzelsträngige DNA oder RNA als auch von doppelsträngiger DNA in B-Konformation in doppelsträngige DNA in Z-Konformation erzielt werden. Diese Konformationsänderungen können in Abhängigkeit von der jeweils eingenommenen Konformation und der Basensequenz des Indikatorbereichs leicht nachgewiesen werden.

Die Konformationsänderung von einzelsträngiger DNA oder RNA in doppelsträngige DNA oder RNA im Indikatorbereich kann z.B. nachgewiesen werden

-durch das Auftreten einer vor der Hybridisierung nicht vorhandenen doppelsträngigen Erkennungssequenz für ein Restriktionsenzym, Schnitt mit diesem Restriktionsenzym und Nachweis des nach bekannten Verfahren radioaktiv oder z.B. mit Biotin markierten Spaltfragments durch bekannte chromatographische oder elektrophoretische Methoden,
-durch das Auftreten einer vor der Hybridisierung nicht vorhandenen doppelsträngigen Erkennungssequenz für ein Repressormolekül, Bindung des Repressors an diese Sequenz, Nachweis des gebundenen Repressors durch spezifische Antikörper gegen den Repressor nach bekannten immunologischen Verfahren oder durch gebundene Repressor-Enzymkonjugate,
-durch das Auftreten einer vor der Hybridisierung nicht vorhandenen doppelsträngigen Promotorsequenz für eine RNA-Polymerase, Polymerisation einzelsträngiger RNA, die nach bekannten Verfahren radioaktiv oder z.B. mit Biotin oder einem Hapten markiert sein kann oder in die ein modifiziertes immunologisch nachweisbares Nucleotid eingebaut werden kann und Nachweis der gebildeten einzelsträngigen RNA durch bekannte chromatographische oder gelelektrophoretische Verfahren oder durch bekannte immunologische Verfahren,

-durch die Bildung einer doppelsträngigen Sequenz, wodurch ein Akzeptor (z.B. Fluorophor) und ein Donor (z.B. Quencher) in derartige Nachbarschaft gebracht werden, daß ein Energietransfer stattfinden kann und ein chemisches oder optisches Signal ausgelöst oder gelöscht wird.

Die Konformationsänderung von doppelsträngiger DNA oder RNA in einzelsträngige DNA oder RNA im Indikatorbereich der Polynucleotidsonde kann z.B. nachgewiesen werden

-durch das Auftreten eines vor der Hybridisierung nicht vorhandenen einzelsträngigen Bereiches, der durch Hybridisierung mit einer zusätzlichen einzelsträngigen, nach bekanntem Verfahren radioaktiv oder z.B. mit Biotin oder einem Hapten markierten DNA-Sonde nachgewiesen wird,
-durch den Abbau des vor der Hybridisierung nicht vorhandenen einzelsträngigen Bereiches durch eine einzelstrangspezifische DNAse oder RNAse und Nachweis der nach bekannten Verfahren radioaktiv oder z.B. mit Biotin oder einem Hapten markierten Abbauprodukte nach bekannten chromatographischen, enzymatischen oder gelelektrophoretischen Verfahren,
-durch das Auftreten eines vor der Hybridisierung nicht vorhandenen einzelsträngigen Bereiches, der als Primer für die Polymerisation durch eine DNA-Polymerase auf einem entsprechenden Template dienen kann und Nachweis der polymerisierten Sequenz durch Lichtstreuung, Bindung basenspezifischer Farbstoffe oder den Einbau eines immunologisch nachweisbaren Nucleotids und anschließenden immunologischen Nachweis,
-durch das Auftreten eines vor der Hybridisierung nicht vorhandenen einzelsträngigen Bereiches definierter Sequenz, der durch Hybridisierung mit einer auf Partikel immobilisierten Probe durch Agglutination oder turbidimetrisch nachgewiesen wird,
-durch das Auftreten eines vor der Hybridisierung nicht vorhandenen einzelsträngigen Bereichs, wodurch ein Akzeptor (z.B. Fluorophor) und ein Donor (z.B. Quencher) in eine räumliche Distanz gebracht werden, so daß kein Energietransfer mehr möglich ist, was zur Auslöschung bzw. Löschung eines detektierbaren chemischen oder optischen Signals führt.

Die Konformationsänderung von doppelsträngiger DNA in B-Konformation in doppelsträngige DNA in Z-Konformation im Indikatorbereich der Polynucleotidsonde kann durch den Einsatz spezifischer Antikörper gegen die durch die Hybridisierung gebildete Z-DNA und den Nachweis der gebundenen Antikörper nach bekannten immunologischen Methoden nachgewiesen werden.

Mit den erfindungsgemäßen Verfahren lassen sich auf einfache Weise Analysenergebnisse erzielen, die den bekannten Verfahren zur Nucleinsäurebestimmung mindestens ebenbürtig sind. Die Verfahren nach der vorliegenden Erfindung können sowohl in Lösung (homogener Assay) als auch mit immobilisierter Nucleinsäure (heterogener Assay) durchgeführt werden. Im ersten Fall entfallen die sonst üblichen Wasch-und Trennungsschritte. Ferner sind die beschriebenen Verfahren auch zur in situ Hybridisierung anwendbar.

Die Sensitivität des Nachweises kann durch den Einsatz verschiedener Polymerasen als Detektionssystem erheblich gesteigert werden, da eine Amplifikation um den Faktor 400-1000 stattfindet.

Die Erfindung wird durch die nachstehenden Beispiele näher erläutert.

Beispiel 1

Herstellung einer radioaktiv markierten Polynucleotidsonde

Eine 53 Nucleotide lange Polynucleotidsonde mit definierter Sequenz wird auf einem DNA-Synthesizer aus Phosphoamiditvorstufen synthetisiert. Nach Beendigung der Synthese wird das Polynucleotid vom Träger abgespalten und nach bekannten chromatographischen Methoden mittels HPLC aufgereinigt. Die Polynucleotidsonde besteht aus einem 19 Nucleotide langen komplementären Bereich und aus einem 20 Nucleotide langen Indikatorbereich, die durch einen Spacer getrennt sind. Die radioaktive Markierung des Polynucleotids erfolgt nach bekannten enzymatischen Methoden, indem auf das 5'OH-Ende des Polynucleotids die terminale Phosphatgruppe von $^{32}P$ ATP durch die T4-DNA-Kinase übertragen wird.

In gleicher Weise kann einer Polynucleotidsonde synthetisiert und radioactiv markiert werden, die keinen Spacer trägt.

Beispiel 2

Nachweis der Konformation im Indikatorbereich der Polynucleotidsonde

15 pmol radioaktiv markierte Polynucleotidsonde werden mit 2 U Restriktionsendonuclease Pst I bei 20 und 37 °C, 30, 60 und 120 Minuten inkubiert. Der Spaltansatz wird auf einem 16 %igen Polyacrylamidgel elektrophoretisiert und die Spaltprodukte durch Autoradiographie sichtbar gemacht.

Die Autoradiographie zeigt, daß unter allen Bedingungen nur die gesamte 53 Nucleotide lange Polynucleotidsonde entsteht. Das bedeutet, daß die Erkennungssequenz für die Restriktionsendonuclease Pst I im Indikatorbereich der Polynucleotidsonde als einzelsträngige DNA vorliegt.

Beispiel 3

Nachweis der Konformationsänderung von einzelsträngiger in doppelsträngige DNA durch Hybridisierung mit M13mp8-DNA

15 pmol radioaktiv markierte Polynucleotidsonde werden 30 Minuten bei 56 °C mit 0, 1, 2, 5, 10, 20 und 50 pmol komplementärer M13mp8-Einzelstrang-DNA inkubiert. Nach Abkühlung auf Raumtemperatur wird mit Restriktionsendonuclease Pst I gespalten und die Ansätze werden auf einem 16 %igen Polyacrylamidgel aufgetrennt. Das Ergebnis zeigt, daß mit wachsender Menge M13mp8-DNA wachsende Mengen des 43 Nucleotide langen Spaltproduktes entstehen.

Das bedeutet, daß durch Hybridisierung mit der komplementären DNA eine Koformationsänderung im Indikatorbereich eintritt, durch die die Spaltsequenz für die Restriktionsendonuclease Pst I entsteht.

Beispiel 4

Nachweis von M13mp8-DNA durch Konformationänderung von doppelsträngiger in einzelsträngige DNA

Zum Nachweis von M13mp8-DNA werden unterschiedliche Mengen M13mp8-DNA mit der Polynucleotidsonde und einer radioaktiv markierten Probe gegen den Indikatorbereich der Polynucleotidsonde unter Hybridisierungsbedingungen inkubiert. Anschließend wird mit der Restriktionsendonuclease Alu I gespalten, das Spaltprodukt über eine DE-52 Minisäule abgetrennt und die Radioaktivität bestimmt.

Das Beispiel zeigt, daß Radioaktivität im Eluat nur in Anwesenheit von M13mp8-DNA auftritt und diese mit er Menge an M13mp8 steigt. Grund dafür ist die Konformationsänderung zu einzelsträngiger DNA im Indikatorbereich der Sonde und die dadurch mögliche Hybridisierung mit der radioaktiv markierten zweiten Sonde zu einer doppelsträngigen DNA, die mit Alu I spaltbar ist.

Beispiel 5

Nachweis von M13mp8-DNA durch Konformationsänderung und eine zweite biotinylierte Sonde

Unterschiedliche Mengen M13mp8-DNA werden in Lösung mit der Polynucleotidsonde unter Hybridisierungsbedingungen inkubiert und anschließend auf Nitrocellulose fixiert. Danach wird mit einer zweiten, zu dem Indikatorbereich der Polynucleotidsonde komplementären Sonde hybridisiert und nicht hybridisierte Sondenmoleküle werden durch Waschen entfernt. Da die zweite Sonde durch Einbau von Biotin-11-dUTP durch die terminale Transferase markiert war, läßt sich die Hybridisierung durch einen Streptavidin-alkalische Phosphatase-Komplex nach bekannten Methoden nachweisen.

Eine blaue Farbreaktion ist nur in den Proben zu beobachten, die mit M13mp8-DNA hybridisiert worden sind. Im Falle der Polynucleotidsonde allein tritt keine Reaktion auf, da die Sonde nicht in einzelsträngiger Form vorliegt und daher nicht mit der zweiten, biotinylierten Sonde hybridisieren kann.

Beispiel 6

Nachweis von M13mp8-DNA durch eine Konformationsänderung von B-DNA zu Z-DNA

M13mp8-DNA wird auf Nitrocellulose fixiert und mit einer Polynucleotidsonde in B-DNA-Konformation hybridisiert. Nach den üblichen Waschschritten wird die Nitrocellulose mit Kaninchen-anti-Z-DNA-Antikörpern inkubiert und anschließend gewaschen. Die gebundenen Antikörper werden durch ein anti-Kaninchen-Peroxidase-Konjugat nach bekannten Methoden nachgewiesen.

Beispiel 7

Nachweis von M13mp8-DNA durch Konformationsänderung von doppelsträngiger in einzelsträngige DNA und anschließende Polymerisation mit einer DNA-Polymerase

Eine Polynucleotidsonde, deren 3'-terminaler Indikatorbereich aus Thymidin-Resten besteht, wird mit M13mp8-DNA in Lösung unter Hybridisierungsbedingungen inkubiert. Anschließend wird poly-dA, Magnesiumchlorid, 2 U Polymerase und $^3$H-dTTP zugesetzt. Nach 30minütiger Inkubation bei 37 °C wird die gebildete Nucleinsäure durch TCA-Fällung präzipitiert und die Menge des eingebauten $^3$H-dTTP im Szintillationszähler bestimmt. Ein deutlicher Einbau von $^3$H-dTTP findet nur im Falle der Hybridisierung mit M13mp8-DNA, nicht aber im Falle von z.B. Kalbsthymus-DNA, Heringssperma oder φX 174-DNA statt. Das zeigt, daß die Thymidin-Reste im Indikatorbereich der Polynucleotidsonde erst nach der Konformationsänderung durch Hybridisierung als Primer für eine Polymerisation dienen können.

Beispiel 8

Nachweis von M13mp8-DNA durch Konformationsänderung und Polymerisation biotinylierter und modifizierter Nucleotide

M13mp8-DNA wird mit der Polynucleotidsonde aus Beispiel 7 wie dort beschrieben hybridisiert. In dem anschließenden Polymerisationsassay wird Biotin-11-dUTP an Stelle von $^3$H-dTTP eingesetzt. Nach der Reaktion wird das gebildete Polybiotin-dU durch einen Streptavidin-Phosphatase-Komplex auf einem Filter immobilisiert und wie in Beispiel 5 beschrieben nachgewiesen.

Anstelle von Biotin-11-dUTP kann in analoger Weise auch Biotin-dUTP eingebaut werden und das Polymerisationsprodukt kann über Biotin-dU-Antikörper-Peroxidase-Konjugate nachgewiesen werden.

**Ansprüche**

1. Verfahren zur Bestimmung von Nucleinsäuren in einer biologischen Probe durch Überführung der zu bestimmenden Nucleinsäuresequenzen in Einzelstränge und Umsetzung dieser Einzelstränge mit einer Polynucleotidsonde, dadurch gekennzeichnet, daß die beim Kontakt der Polynucleotidsonde, bestehend aus einem zur nachzuweisenden Nucleinsäure komplementären Bereich und einem Indikatorbereich, mit der nachzuweisenden Nucleinsäurespezies unter Hybridisierungsbedingungen entstehende Konformationsänderung im Indikatorbereich nachgewiesen wird.

2. Verfahren nach Anspruch 1, dadurch gekennzeichnet, daß der zur nachzuweisenden Nucleinsäure komplementäre Bereich und der Indikatorbereich der Polynucleotidsonde sich überlappen, nicht überlappen oder durch einen Spacer getrennt sind.

3. Verfahren nach den Ansprüchen 1 und 2, dadurch gekennzeichnet, daß durch die Hybridisierung im Indikatorbereich eine Konformationsänderung von einzelsträngiger DNA (RNA) in doppelsträngige DNA (RNA), von doppelsträngiger DNA (RNA) in einzelsträngige DNA (RNA) oder von doppelsträngiger DNA in B-Konformation in doppelsträngige DNA in Z-Konformation erfolgt.